# EUROPEAN PATENT SPECIFICATION

(11) **EP 4 278 964 B1**
(45) Date of publication and mention of the grant of the patent: **28.05.2025**
(21) Application number: 22749796.3
(22) Date of filing: 03.02.2022
(51) Int. Cl.: A61B 5/055, A61B 6/03, G06T 1/00

(54) **INFORMATION PROCESSING SYSTEM, PROGRAM, AND INFORMATION PROCESSING METHOD**
INFORMATIONSVERARBEITUNGSSYSTEM, PROGRAMM UND INFORMATIONSVERARBEITUNGSVERFAHREN
SYSTÈME DE TRAITEMENT D'INFORMATIONS, PROGRAMME, ET PROCÉDÉ DE TRAITEMENT D'INFORMATIONS

(30) Priority: 04.02.2021 JP 2021016358
(43) Date of publication of application: 22.11.2023
(73) Proprietor: KOMPATH, INC., Tokyo 1130033 (JP); THE UNIVERSITY OF TOKYO, Tokyo 1138654 (JP)
(72) Inventor: DOKE, Takehito, Tokyo 113-0033 (JP); SAITO, Toki, Tokyo 113-8654 (JP); KIN, Taichi, Tokyo 113-8654 (JP); OYAMA, Hiroshi, Tokyo 113-8654 (JP); SAITO, Nobuhito, Tokyo 113-8654 (JP); KIYOFUJI, Satoshi, Tokyo 113-8654 (JP)
(74) Representative: Hasegawa, Kan
(86) International application number: PCT/JP2022/004318
(87) International publication number: WO 2022/168925

(56) References cited:
- WO-A1-2011/010644
- WO-A1-2017/138558
- CN-A- 112 150 419
- JP-A- 2005 202 791
- JP-A- 2007 135 858
- JP-A- 2010 017 421
- JP-A- 2012 088 771
- JP-A- 2013 222 361
- JP-A- 2016 087 359
- AL-REI MONA: "AUTOMATED 3D VISUALIZATION OF BRAIN CANCER", THESIS, 1 June 2017 (2017-06-01), pages 1 - 138, XP055921553

## Description

### TECHNICAL FIELD

The present invention relates to an information processing system, a program, and an information processing method.

### RELATED ART

In recent years, techniques of three-dimensional computer graphics for reconstructing sequential sectional images of three-dimensional objects have been developed. For example, Patent Document 1 discloses a technique of acquiring color information by performing volume rendering processing on a three-dimensional image and assigning, based on the acquired color information, colors to a surface of surface data generated from the three-dimensional image.

### PRIOR ART DOCUMENTS

### Patent document

[Patent Document 1] PCT International Application, Laid-Open No. 2018/159709
AL-REI MONA: "AUTOMATED 3D VISUALIZATION OF BRAIN CANCER", THESIS, 1 June 2017 (2017-06-01), pages 1-138, describes an automated 3D visualization system for brain cancer, called M-3Ds, which processes 2D DICOM images to segment brain tumors to isolate the tumor from surrounding tissues and generate interactive 3D models.
CN 112 150 419 A describes a tissue imaging process that involves receiving an input image sequence comprising at least two image sequences. A reference image sequence is determined, and other image sequences are registered based on this reference. All registered sequences are converted into the same image space. Pixel values are modified based on tissue segmentation results and preset numerical ranges. Finally, the processed intermediate image sequences are fused to create an output image sequence.
JP 2007 135858 A describes a bone region imaging process involving the following steps: A reference tomographic image is selected, and a bone extraction region is calculated by excluding air regions from the internal body region. Next, the bone region is identified through region expansion processing from pixels indicating bone. Finally, bone-removed images are created by subtracting the bone regions from the tomographic images, and a 3D image of the subject without bones is reconstructed and displayed.
JP 2016 087359 A describes a method for creating a biological texture model involving the following steps: Obtain two-dimensional distribution data of CT values from cross-sectional images. Prepare minimum basic units with different hardness levels and obtain their mechanical property data. Estimate bone tissue regions from the CT values. Calculate bone density from the CT values and estimate mechanical property data for each pixel. Assign the most similar minimum basic unit to each pixel. Layer the cross-sectional images to generate 3D data and create the model using a 3D printer.

### SUMMARY OF INVENTION

### Problems to be Solved by Invention

However, the technique disclosed in Patent Document 1 could not sufficiently reproduce a microscopic shape of three-dimensional objects having complex three-dimensional structures such as human body tissue.

In view of the above circumstances, the present invention provides a technique capable of visualizing a microscopic shape and representing it in a three-dimensional image.

### Means for Solving Problems

According to an aspect of the present invention, an information processing system according to claim 1 is provided.

According to such an aspect, a microscopic shape can be visualized and represented in a three-dimensional image.

### BRIEF DESCRIPTION OF DRAWINGS

FIG. 1 is a block diagram illustrating a hardware configuration of an information processing apparatus 1.
FIG. 2 is a block diagram illustrating functions realized by a controller 13 and the like in the information processing apparatus 1 according to the first embodiment.
FIG. 3 is an activity diagram illustrating a flow of information processing executed by the information processing apparatus 1.
FIG. 4 is a diagram illustrating a plurality of MRI images 222 and one MRI image 212.
FIG. 5 is a diagram illustrating a contour portion 213 of a whole brain area in the MRI image 212.
FIG. 6 is a diagram illustrating an example of generated three-dimensional data 230.
FIG. 7 is a diagram illustrating a setting screen 300 for setting material information for a whole brain area.
FIG. 8 is a diagram making a comparison between three-dimensional data without material information and three-dimensional data with material information.
FIG. 9 is a diagram making a comparison between three-dimensional data without material information and three-dimensional data with material information.
FIG. 10 is a diagram making a comparison between three-dimensional data without material information and three-dimensional data with material information.
FIG. 11 is a diagram making a comparison between three-dimensional data without material information and three-dimensional data with material information.
FIG. 12 is an activity diagram illustrating a flow of information processing executed by the information processing apparatus 1.
FIG. 13 is a diagram illustrating a plurality of CT images 220 and one CT image 210.
FIG. 14 is a diagram making a comparison between three-dimensional data without a correction process and three-dimensional data with a correction process.
FIG. 15 is a diagram making a comparison between (partial) three-dimensional data without shape information based on a material and (partial) three-dimensional data with shape information based on a material.
FIG. 16 is a diagram illustrating a material application image 231 in which material information is set for a whole predetermined area (in FIG. 16, the whole brain) on an arbitrary one of the plurality of MRI images 222.

### DETAILED DESCRIPTION OF EMBODIMENTS

Hereinafter, embodiments of the present invention will be described with reference to drawings. Various features described in the following embodiments can be combined with each other.

A program for realizing a software described in the present embodiment may be provided as a computer-readable non-transitory medium, may be provided to be downloaded via an external server, or may be provided so that the program is activated on an external computer and its function is realized on a client terminal (so-called cloud computing).

A term "unit" in the present embodiment may include, for example, a combination of hardware resources implemented as circuits in a broad sense and information processing of software that can be concretely realized by the hardware resource. Furthermore, various types of information are described in the present embodiment, and such information may be represented by, for example, physical values of signal values representing voltage and current, high and low signal values as a set of binary bits consisting of 0 or 1, or quantum superposition (so-called qubits), and communication and computation may be executed on a circuit in a broad sense.

The circuit in a broad sense is a circuit realized by properly combining at least a circuit, circuitry, a processor, a memory, and the like. In other words, a circuit includes an application specific integrated circuit (ASIC), a programmable logic device (e.g., simple programmable logic device (SPLD), a complex programmable logic device (CLPD), field programmable gate array (FPGA), and the like.

### 1. Hardware Configuration

The present section describes a hardware configuration according to the first embodiment. In the present embodiment, an information processing system includes one or more apparatuses or components. Thus, for example, even an information processing apparatus 1 alone is an example of an information processing system. Hereinafter, a description is given of a hardware configuration of the information processing apparatus 1 as an example of an information processing system.

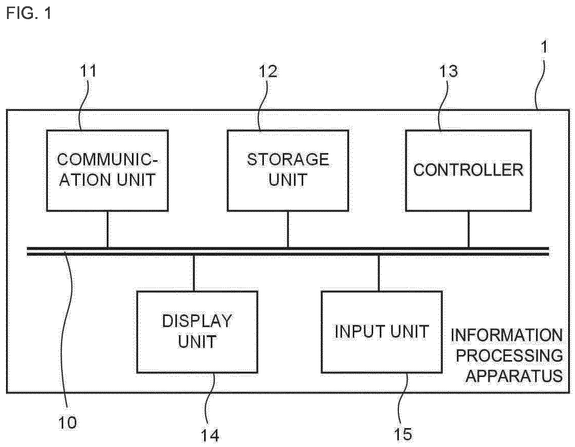

FIG. 1 is a block diagram illustrating the hardware configuration of the information processing apparatus 1. The information processing apparatus 1 includes a communication unit 11, a storage unit 12, a controller 13, a display unit 14, and an input unit 15, and these components are electrically connected via a communication bus 10 in the information processing apparatus 1. Each component will be further described.

The communication unit 11 may be wired communication means such as USB, IEEE1394, Thunderbolt, wired LAN network communication, and the like, but may include wireless LAN network communication, mobile communication such as 3G/LTE/5G, Bluetooth (registered trademark) communication, and the like as needed. The communication unit 11 may be implemented as a set of two or more of these communication means. In other words, the information processing apparatus 1 may communicate various types of information with an external device via the communication unit 11 and a network.

The storage unit 12 stores various types of information as defined by the above description. The storage unit 220 may be implemented, for example, as a storage device such as a solid state drive (SSD) storing various programs, etc. pertaining to the information processing apparatus 1 and executed by the controller 13, or as a memory such as a random access memory (RAM) storing temporarily necessary information (arguments, sequences, etc.) pertaining to program operations. The storage unit 12 stores various programs, variables, etc. pertaining to the information processing apparatus 1 and executed by the controller 13.

The controller 13 executes processing of and provides control on an overall operation relating to the information processing apparatus 1. The controller 13 is, for example, an unshown central processing unit (CPU). The controller 13 realizes various functions pertaining to the information processing apparatus 1 by reading a predetermined program stored in the storage unit 12. That is, information processing by software stored in the storage unit 12 is concretely realized by the controller 13 as an example of hardware and thereby may be executed as each functional unit included in the controller 13. A more detailed description thereof will be described in the next section. The controller 13 is not limited to consisting of a single controller but may be implemented so that two or more controllers 13 for respective functions are included. Alternatively, the controller 13 may be a combination thereof.

The display unit 14 may be, for example, included in a housing of the information processing apparatus 1 or may be externally provided. The display unit 14 displays a graphical user interface (GUI) screen operable by a user. Depending on a type of information processing apparatus 1, the display unit 14 may be differently implemented as a display device such as, for example, a CRT display, a liquid crystal display, an organic EL display, and a plasma display.

The input unit 15 may be included in the housing of the information processing apparatus 1 or may be externally provided. For example, the input unit 15 may be implemented as a touch panel integrated with the display unit 14. When the input unit 15 is a touch panel, the user can make input by a tapping operation, a swiping operation, or the like. The input unit 15 may be a switch button, a mouse, a QWERTY keyboard, or the like instead of a touch panel. That is, the input unit 15 receives operation input made by the user. The input is transferred as an instruction signal to the controller 13 via the communication bus 10, and the controller 13 may execute predetermined control or operation as necessary.

### 2. Functional Configuration

The present section describes a functional configuration according to the first embodiment. As described above, information processing by the software stored in the storage unit 12 is concretely realized by the controller 13 as an example of hardware, and thereby each functional unit included in the controller 13 may be executed.

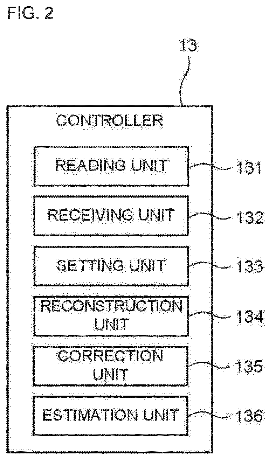

FIG. 2 is a block diagram illustrating functions realized by the controller 13 or the like in the information processing apparatus 1 according to the first embodiment. Specifically, the information processing apparatus 1 includes the controller 13. The controller 13 includes a reading unit 131, a receiving unit 132, a setting unit 133, a reconstruction unit 134, a correction unit 135, and an estimation unit 136.

The reading unit 131 is configured to read various types of information received from an external device via the communication unit 11 or stored in advance in the storage unit 12.

The receiving unit 132 is configured to receive various types of information. The receiving unit 132 as an example of a first receiving unit is configured to receive first operation input made by the user with respect to sequential sectional images. The receiving unit 132 as an example of a second receiving unit is configured to receive second operation input made by the user with respect to the sequential sectional images. The receiving unit 132 as an example of a third receiving unit is configured to receive third operation input made by the user with respect to the sequential sectional images.

The setting unit 133 is configured to set material information representing a material of an object.

The reconstruction unit 134 is configured to reconstruct a plurality of sequential sectional images including a predetermined area for which material information is set and to generate three-dimensional data on the object.

The correction unit 135 is configured to execute a correction process on various types of information. For example, the correction unit 135 executes the correction process on at least one of first data including material information on the object and second data including a shape of the object.

The estimation unit 136 is configured to estimate various types of information. For example, based on the material information, the estimation unit 136 estimates shape information on a detailed shape of the object.

### 3. Information Processing Method

This section describes an information processing method in the above-described information processing apparatus 1. This information processing method is an information processing method executed by a computer. The information processing method includes a reading step, a setting step, and a reconstruction step. The reading step reads a plurality of sequential sectional images of an object. Based on a pixel value of a pixel in a predetermined area included in the sequential sectional images and preset reference information, the setting step sets, for the predetermined area, material information representing a material of the object. Here, the reference information is information where a pixel value and a material are associated with each other. The reconstruction step reconstructs the plurality of sequential sectional images including the predetermined area for which the material information is set and thereby generates three-dimensional data on the object.

### 3.1 Overview of Information Processing Method

The information processing system includes the controller 13. The controller 13 includes each of the following units. The reading unit 131 reads a plurality of sequential sectional images of an object. Based on a pixel value of a pixel in a predetermined area included in the sequential sectional images and preset reference information, the setting unit 133 sets, for the predetermined area, material information representing a material of the object. The reference information is information where a pixel value and a material are associated with each other. The reconstruction unit 134 reconstructs the plurality of sequential sectional images including the predetermined area for which the material information is set and thereby generates three-dimensional data on the object.

The sequential sectional images may include a first medical image captured by a first medical image diagnosis apparatus and a second medical image captured by a second medical image diagnosis apparatus. Based on the first medical image, the setting unit 133 sets the material information. The reconstruction unit 134 reconstructs the first medical image and generates first data including the material information on the object. The reconstruction unit 134 reconstructs the second medical image and generates second data including a shape of the object. Based on the first data and the second data, the reconstruction unit 134 generates three-dimensional data.

Furthermore, the correction unit 135 may execute a correction process on at least one of the first data and the second data. Based on the first data and the second data after the correction, the reconstruction unit 134 generates three-dimensional data.

Based on the material information, the estimation unit 136 may further estimate shape information on a detailed shape of the object. Based on the shape information, the reconstruction unit generates three-dimensional data.

The first medical image diagnosis apparatus may be a magnetic resonance imaging apparatus. The second medical image diagnosis apparatus may be an X-ray computed tomography apparatus.

The reconstruction unit 134 may determine, based on the material information, color viewed when the three-dimensional data is displayed and may generate three-dimensional data.

The sequential sectional images may be medical images. In this aspect, the object is predetermined tissue or organ of a human body.

Based further on a type of the medical image, the setting unit 133 may set the material information. Here, the reference information is information where a type of the medical image, a pixel value, and a material are associated with each other.

In the reference information, one material may be associated with one pixel value.

Based on a pixel value of a pixel in a contour portion of the predetermined area and the reference information, the setting unit 133 may set the material information for the contour portion.

The setting unit 133 may select, depending on the predetermined area, reference information from two or more pieces of preset reference information and set, based on the reference information, material information.

Furthermore, the receiving unit 132 as an example of the first receiving unit may receive first operation input made by the user with respect to the sequential sectional images. The first operation input includes information selecting one from two or more pieces of preset reference information. The setting unit 133 sets the material information based on the first operation input.

Furthermore, the receiving unit 132 as an example of a second receiving unit may receive a second operation input made by the user with respect to the sequential sectional images. The second operation input includes information specifying the predetermined area.

Furthermore, the receiving unit 132 as an example of a third receiving unit may receive third operation input made by the user with respect to the sequential sectional images. The third operation input may include information specifying color corresponding to the material information. Based on the material information and the third operation input, the reconstruction unit 134 may generate three-dimensional data on the object.

An aspect of the present invention may be a program. The program allows a computer to execute each step of the information processing system.

### 3.2 Details of Information Processing Method

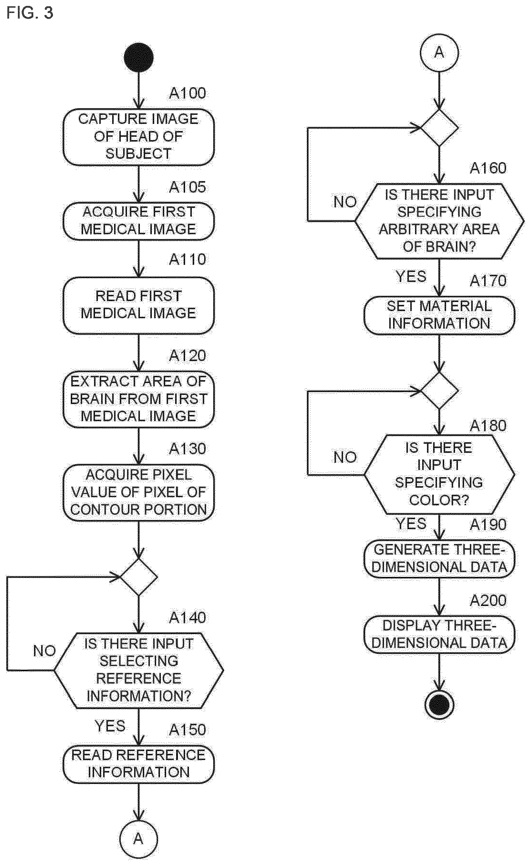

FIG. 3 is an activity diagram illustrating a flow of information processing executed by the information processing apparatus 1. The following description follows each activity in this activity diagram.

In the first embodiment, for convenience of explanation, each term will be used as described below. Sequential sectional images are medical images conforming to a DICOM (Digital Imaging and Communications in Medicine) standard and includes, in particular, a first medical image captured by a first medical image diagnosis apparatus. The first medical image diagnosis apparatus is a magnetic resonance imaging apparatus. The first medical image means an image captured by the magnetic resonance imaging apparatus (hereinafter referred to as "MRI image 212"). The object is predetermined tissue or organ of a human body, and means, in particular, a brain. The predetermined area means a whole brain area including a cerebrum, a cerebellum, and a brainstem.

The magnetic resonance imaging apparatus captures an image of a head of a subject (Activity A100). The magnetic resonance imaging apparatus includes an unshown transmit coil and an unshown receive coil. The transmit coil excites an arbitrary area of the subject (in the present embodiment, the head of the subject) by applying a high frequency magnetic field. The transmit coil is disposed so that, for example, the transmit coil surrounds the head of the subject. The transmit coil receives a supply of RF pulses from an unshown transmit circuit, generates a high-frequency magnetic field, and applies the high-frequency magnetic field to the subject. The transmit circuit supplies the RF pulses to the transmit coil under control of an unshown sequence control circuit.

The receive coil is located on an inner side of an unshown gradient coil and receives a magnetic resonance signal (hereinafter, referred to as "MR (Magnetic Resonance) signal") emitted from a subject due to an effect of the high-frequency magnetic field. When the MR signals are received, the received MR signals are output to a receive circuit.

The receive circuit generates MR data by performing analog-to-digital (AD) conversion on the analog MR signals output from the receive coil. The receive circuit transmits the generated MR data to the unshown sequence control circuit. In this way, a plurality of MRI images 222 as sequential sectional images are acquired.

Subsequently, the generated plurality of MRI images 222 are imported to the information processing apparatus 1 via communication network. In other words, in the information processing apparatus 1, the controller 13 acquires the plurality of MRI images 222 having three-dimensional information on the head of the subject or capable of restoring three-dimensional information on the head of the subject via the communication unit 11 (Activity A105). The controller 13 allows the storage unit 12 to store the acquired plurality of MRI images 222.

The controller 13 executes a reading step (Activity A110). In the reading step, the reading unit 131 reads the plurality of MRI images 222 pertaining to the brain. That is, for example, the controller 13 executes a process of reading the plurality of MRI images 222 pertaining to the brain stored in the storage unit 12.

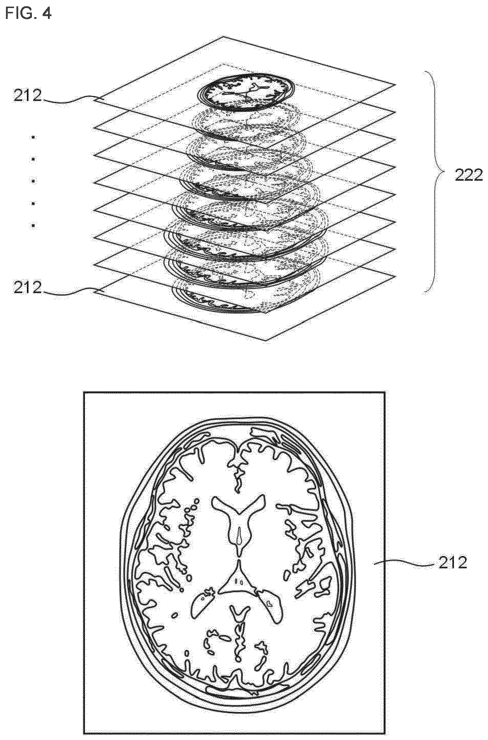

FIG. 4 is a diagram illustrating a plurality of MRI images 222 and one MRI image 212. The MRI image 212 is one sectional brain image of the plurality of MRI images 222 acquired by capturing an image of the head of the subject with the magnetic resonance imaging apparatus. In this information processing method, three-dimensional data 230 is generated and displayed by executing various types of information processing on the plurality of MRI images 222 (each MRI image 212).

Subsequently, the controller 13 executes a process of extracting a whole brain area from the MRI image 212 and allows the storage unit 12 to store data on the extracted whole brain area (Activity A120). The process of extracting the whole brain area is performed by, for example, by executing a known segmentation process on the MRI image 212.

Subsequently, the controller 13 acquires a pixel value of a pixel in a contour portion 213 of the whole brain area extracted from the MRI image 212 (Activity A130). That is, Activity A130 includes, for example, the following four steps of information processing. (1) The controller 13 reads data on a whole brain area in the MRI image 212, the data having been stored in the storage unit 12. (2) By executing a known segmentation process, the controller 13 identifies a contour portion 213 of the whole brain area. (3) The controller 13 acquires a pixel value of a pixel in the identified contour portion 213. (4) The controller 13 allows the storage unit 12 to store data on the acquired pixel value of the pixel. The controller 13 executes the above processes (1) to (4) for every MRI image 212.

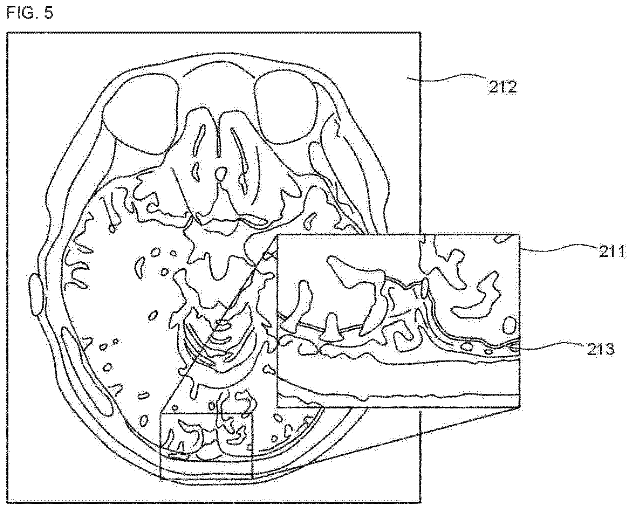

FIG. 5 is a diagram illustrating the contour portion 213 of the whole brain area in the MRI image 212. By executing the process in Activity A130, the controller 13 identifies the contour portion 213 represented in a contour image 211 and acquires the pixel values of the pixels of the contour portion 213.

Subsequently, the controller 13 executes a first receiving step (Activity A140). In the first receiving step, the receiving unit 132 receives first operation input made by the user with respect to the MRI image 212. Here, the first operation input includes information selecting one from two or more pieces of preset reference information. That is, activity A140 includes, for example, the following three steps of information processing. (1) The input unit 15 receives first operation input made by the user. (2) The input unit 15 allows the first operation input to be transferred, to the controller 13, as an instruction signal via the communication bus 10. (3) The controller 13 receives the transferred instruction signal pertaining to the first operation input.

Subsequently, based on the first operation input executed in Activity A140, the controller 13 reads the reference information stored in the storage unit 12 (Activity A150). Here, the reference information is information where a type of a medical image, a pixel value, and material are associated with each other, and is information where one material is associated with one pixel value. In the present embodiment, the type of the medical image is MRI image. Therefore, the reference information is two or more templates supporting the MRI image. A description of the templates will be given below. A type of an object that can be easily extracted is different depending on the type of the medical image. For example, MRA images acquired by a time-of-flight (TOF) method represents blood vessels well, and therefore when the type of the medical image is MRA image acquired by the TOF method, reference information with the material of the blood vessel set may be read. A material means, according to the invention, is a parameter of at least one of albedo (reflectivity to each of RGB (red, green, and blue) light), reflectance (metallic characteristic), roughness.

Subsequently, the controller 13 executes a second receiving step (Activity A160). In the second receiving step, the receiving unit 132 receives second operation input made by the user with respect to the MRI image 212. Here, the second operation input includes information specifying an arbitrary area of the brain included in the MRI image 212. In other words, activity A160 includes, for example, the following three steps of information processing. (1) The input unit 15 receives second operation input made by the user. (2) The input unit 15 allows the second operation input to be transferred, to the controller 13, as an instruction signal via the communication bus 10. (3) The controller 13 receives the transferred instruction signal pertaining to the second operation input. The arbitrary area is, for example, cerebrum, cerebellum, brainstem, or the like. In the present embodiment, all areas of the brain are specified.

Subsequently, the controller 13 executes a setting step (Activity A170). In the setting step, based on the type of the medical image (MRI image in the present embodiment ), the pixel value of the pixel in the contour portion 213 of the whole brain area included in the MRI image 212, and the reference information preset by the first operation input, the setting unit 133 sets material information on a material of the brain for the contour portion 213 of the whole area of the brain. (Activity A170).

In other words, Activity A170 includes, for example, the following four steps of information processing. (1) The controller 13 reads data stored in the storage unit 12 and representing that the MRI image 212 is an image captured by a magnetic resonance imaging apparatus. (2) The controller 13 reads data on the extracted whole brain area, the data having been stored in the storage unit 12. (3) The controller 13 executes a setting process on the reference information read in Activity A150 and on the data read in (1) and (2) above. (4) The controller 13 sets material information for the contour portion 213 of the whole brain area in the MRI image 212.

The material information is, according to the invention, information on each parameter of at least one of albedo (reflectivity to each of RGB (red, green, and blue) light), reflectance (metallic characteristic), and roughness, as described above. As material information on brains and blood vessels, a shiny material may be set so that wet surfaces can be represented. As material information on bones, a matte material may be set so that a dry surface can be represented.

Subsequently, the controller 13 executes a third receiving step (Activity A180). In the third receiving step, the receiving unit 132 receives third operation input made by the user with respect to the MRI image 212. Here, the third operation input includes information specifying color corresponding to the material information representing the material of the brain. In other words, Activity A180 includes the following three steps of information processing. (1) The input unit 15 receives third operation input made by the user. (2) The input unit 15 allows the third operation input to be transferred, to the controller 13, as an instruction signal via the communication bus 10. (3) The controller 13 receives the transferred instruction signal pertaining to the third operation input.

Subsequently, the controller 13 executes a reconstruction step (Activity A190). In the reconstruction step, the reconstruction unit 134 reconstructs the plurality of MRI images 222 including the whole brain area for which the material information is set and, based on the material information and the third operation input, generates three-dimensional data 230 on the brain. In other words, the controller 13 generates colored three-dimensional data 230 on the brain by reading the plurality of MRI images 222 stored in the storage unit 12 and executing the reconstruction process. The controller 13 allows the storage unit 12 to store the generated three-dimensional data 230. The three-dimensional data 230 corresponds to the plurality of MRI images 222 with texture applied, and the three-dimensional data 230 having an increased information amount can be effectively used as medical data.

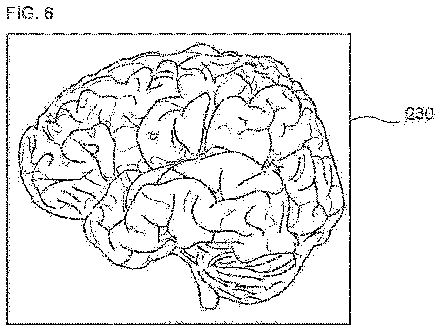

FIG. 6 is a diagram illustrating an example of the generated three-dimensional data 230. The controller 13 generates the three-dimensional data 230 by executing, in the above-described activities A110 to A190, a known segmentation process on the MRI image 212 and thereafter executing a rendering process. Based on the set material information, i.e., without referring to a pixel value of the image, the controller 13 executes the rendering process.

Subsequently, the controller 13 allows the display unit 14 to display the three-dimensional data 230 on the brain (Activity A200). By reading the three-dimensional data 230 on the brain stored in the storage unit 12 and executing a display process, the controller 13 allows the display unit 14 to display the three-dimensional data 230 on the brain.

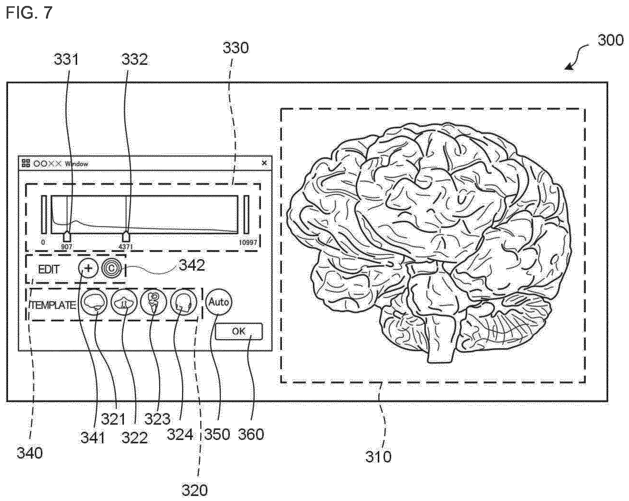

FIG. 7 is a diagram illustrating a setting screen 300 for setting material information for the whole brain area. The setting screen 300 includes a three-dimensional data generation area 310, a template area 320, a material setting area 330, an edit area 340, an auto button 350, and an OK button 360.

The three-dimensional data generation area 310 is an area for displaying the three-dimensional data 230 when various types of information such as material information is applied to the whole brain area extracted from the plurality of MRI images 222 (each MRI image 212).

The template area 320 is an area for displaying a template suitable for the type of the medical image (MRI image in the present embodiment). In the template area 320, a template 321, a template 322, a template 323, and a template 324 are displayed as examples of templates. The template 321 is a template applied to cerebrums. The template 322 is a template applied to cerebellums. The template 323 is a template applied to brainstems. The template 324 is a template applied to skin. In the templates, materials that can be applied to respective parts are set.

The material setting area 330 is an area where color to be applied is specified by sliding a slider 331 and a slider 332. The horizontal axis of the material setting area 330 represents pixel value, and the vertical axis of the material setting area 330 represents brightness of color. For example, when the slider 331 is slid to a pixel value of 907 and the slider 332 is slid to a pixel value of 4371, milky white can be set for a pixel in a contour portion with a pixel value less than 907, black brown can be set for a pixel in the contour portion with a pixel value of 4371 or more, and linearly interpolated color can be set for a pixel in the contour portion with a pixel value of 907 or more and less than 4371.

The edit area 340 includes an addition button 341 and a clear button 342. The addition button 341 is configured to allow addition of a template other than the templates displayed in the template area 320 when being clicked. The clear button 342 is configured to clear various settings and return settings to default settings when being clicked.

The auto button 350 automatically sets a material when being clicked. The OK button 360 starts the rendering process according to the current settings when being clicked.

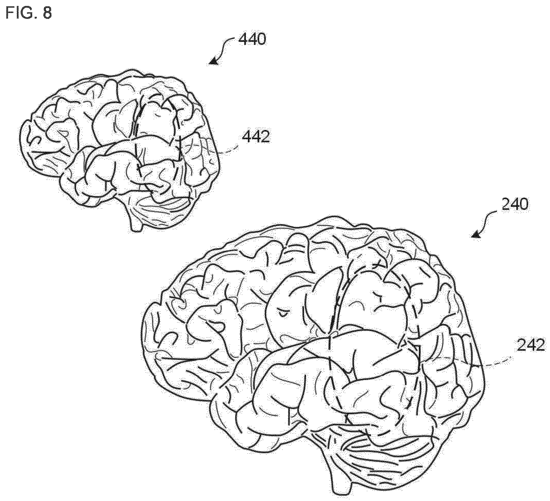

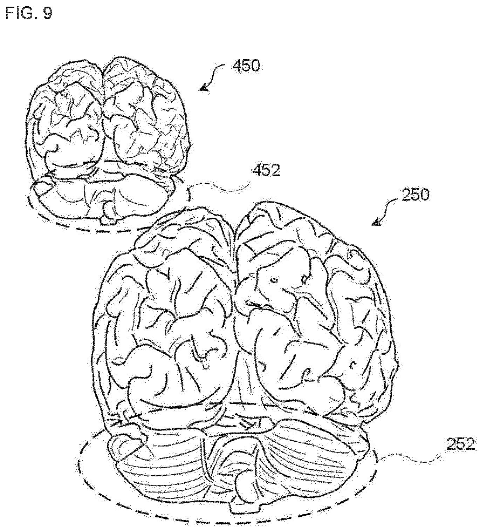

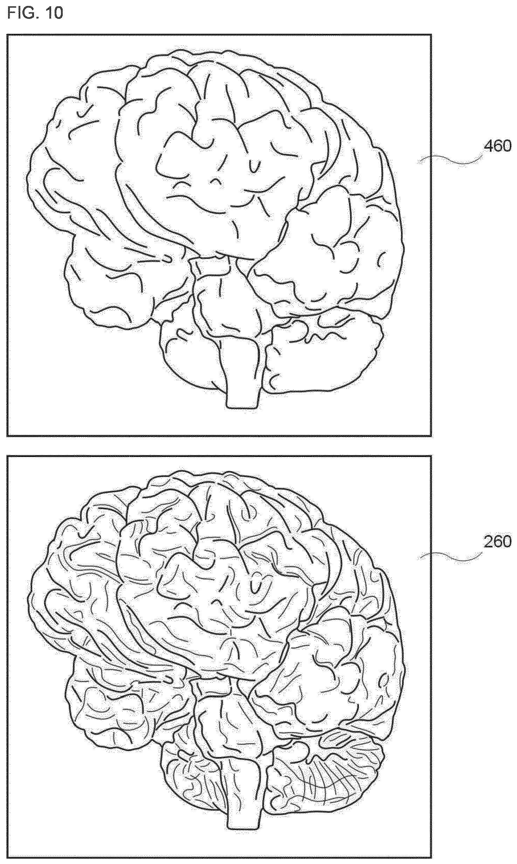

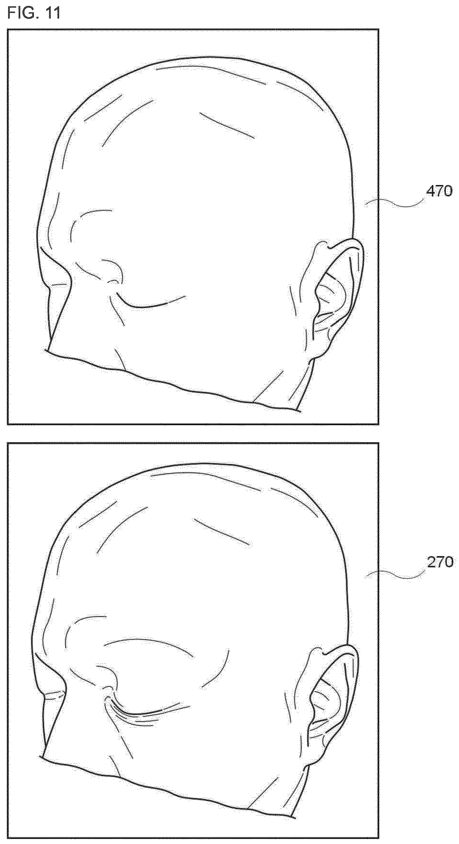

Each of FIG. 8 to FIG. 11 is a diagram making a comparison between three-dimensional data without material information and three-dimensional data with material information. In FIG. 8, for example, shapes of cerebral wrinkles are indistinct and are not visualized in an area 442 in a comparative example 440 without material information, but shapes of cerebral wrinkles are visualized well in an area 242 in an example 240 with material information. In FIG. 9, for example, shapes of cerebellar wrinkles are indistinct and is not visualized in an area 452 in a comparative example 450 without material information, but shapes of cerebellar wrinkles are visualized well in an area 252 in an example 250 with material information. In FIG. 10, an example 260 with material information can visualize minute shape changes of a whole brain better than a comparative example 460 without material information. In FIG. 11, an example 270 with material information can visualize, for example, loosening shapes around eyes better than a comparative example 470 without material information.

According to the above, visualizing pixel value changes by adding material information allows minute shapes to be visualized and to be represented in three-dimensional images. In other words, according to the present embodiment, since one-dimensional information in the form of pixel values acquired from a plurality of sequential sectional images of an object is developed into multidimensional information in the form of material information set based on two or more parameters, and this multidimensional information (material information) is applied to the original sequential sectional images, it is possible to visualize minute shapes and to represent the minute shapes in a three-dimensional image.

In the present embodiment, the sequential sectional images are medical images, and the object is predetermined tissue or organ of a human body. According to such an aspect, it is possible to more realistically represent human body tissue or organ having a complex shape and to make it useful for medical treatment.

In the present embodiment, based further on a type of a medical image, the setting step sets material information, and reference information is information where a type of a medical image, a pixel value, and a material are associated with each other. According to such an aspect, it is possible to visualize microscopic shapes more suitably for characteristics of the type of the medical image.

In the present embodiment, in the reference information, one material is associated with one pixel value. According to such an aspect, an associated relationship between the pixel value and the material can be clarified.

In the present embodiment, based on a pixel value of a pixel in a contour portion of a predetermined area and reference information, material information is set for the contour portion. According to such an aspect, it is possible to visualize an intricate structure of a contour portion of a brain, etc.

In the present embodiment, the first receiving step receives first operation input made by the user with respect to sequential sectional images, the first operation input includes information selecting one from two or more pieces of preset reference information, and based on the first operation input, the setting step sets material information. According to such an aspect, microscopic shapes can be visualized more properly by allowing the user to exert the user's insight and determination.

In the present embodiment, the second receiving step receives second operation input made by the user with respect to sequential sectional images, and the second operation input includes information specifying a predetermined area. According to such an aspect, it is possible to more realistically represent part that the user wants to check in detail (e.g., cerebellum part of a whole brain).

In the present embodiment, the third receiving step receives third operation input made by the user with respect to sequential sectional images, the third operation input includes information specifying color corresponding to material information, and based on the material information and the third operation input, the reconstruction step generates three-dimensional data on the object. According to such an aspect, usability can be improved.

### 4. Second Embodiment

This section describes an information processing apparatus 1 according to the second embodiment. A description will be omitted of functions and configurations substantially similar to those in the first embodiment.

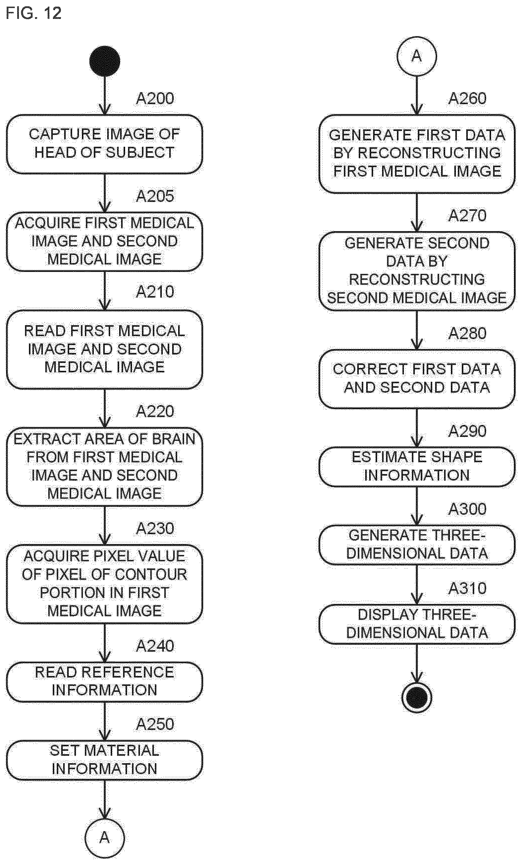

FIG. 12 is an activity diagram illustrating a flow of information processing executed by the information processing apparatus 1. The following description follows each activity in this activity diagram.

In the second embodiment, for convenience of explanation, each term will be used as follows. Sequential sectional images are medical images conforming to the DICOM (Digital Imaging and Communications in Medicine) standard and includes, in particular, a first medical image captured by a first medical image diagnosis apparatus and a second medical image captured by a second medical image diagnosis apparatus. The first medical image diagnosis apparatus is a magnetic resonance imaging apparatus. The second medical image diagnosis apparatus is an X-ray computed tomography apparatus. The first medical image means an image captured by the magnetic resonance imaging apparatus (hereinafter referred to as "MRI image 212"). The second medical image means an image captured by the X-ray computed tomography apparatus (hereinafter referred to as "CT image 210"). The object is predetermined tissue or organ of a human body, and is, in particular, a brain. A predetermined area means a whole brain area.

The X-ray computed tomography apparatus and the magnetic resonance imaging apparatus captures images of a head of a subject (Activity A200). The imaging principle of the magnetic resonance imaging apparatus is as described in Activity A100. In the X-ray computed tomography apparatus, an X-ray detector detects X-rays emitted from an unshown X-ray tube and outputs detected data corresponding to an amount of the X-rays as electrical signals to an unshown DAS (data acquisition system). Then, by rotating, around the subject, an unshown rotation frame supporting the X-ray tube and the X-ray detector facing each other, detection data is collected for a plurality of views, i.e., a whole circumference of the subject. In this way, a plurality of CT images 220 as sequential sectional images are acquired.

Next, the generated plurality of CT images 220 and plurality of MRI images 222 are imported to the information processing apparatus 1 via communication network. In other words, in the information processing apparatus 1, the controller 13 acquires, via the communication unit 11, the plurality of CT images 220 and the plurality of MRI images 222 having three-dimensional information on the head of the subject or capable of restoring three-dimensional information on the head of the subject (Activity A205). The controller 13 allows the storage unit 12 to store the acquired plurality of CT images 220 and plurality of MRI images 222.

The controller 13 executes a reading step (Activity A210). In the reading step, the reading unit 131 reads the plurality of CT images 220 and plurality of MRI images 222 pertaining to the brain. That is, the controller 13 executes a process of reading the plurality of CT images 220 and plurality of MRI images 222 pertaining to the brain and stored in the storage unit 12.

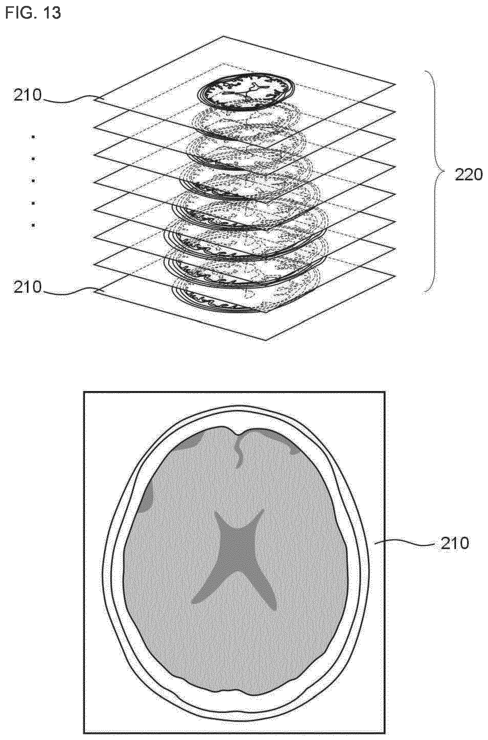

FIG. 13 is a diagram illustrating the plurality of CT images 220 and one CT image 210. The plurality of MRI images 222 and the MRI image 212 are as illustrated in FIG. 4. The CT image 210 is a sectional brain image of one of the plurality of CT images 220 acquired by capturing images of the head of the subject with the X-ray computed tomography apparatus. In the information processing method according to the present embodiment, three-dimensional data 230 is generated and displayed by executing various types of information processing on the plurality of CT images 220 (each CT image 210) and the plurality of MRI images 222 (each MRI image 212).

Subsequently, the controller 13 executes a process of extracting a whole brain area from the CT image 210 and the MRI image 212 and allows the storage unit 12 to store data on the extracted whole brain area (the area extracted from the CT image 210 and the area extracted from the MRI images 212) (Activity A220). The process of extracting the whole brain area is performed by, for example, executing a known segmentation process on the CT image 210 and the MRI image 212.

Subsequently, the controller 13 acquires a pixel value of a pixel in a contour portion of the whole brain area extracted from the MRI image 212 (Activity A230). That is, Activity A230 includes, for example, the following four steps of information processing. (1) The controller 13 reads data on the whole brain area in the MRI image 212, the data having been stored in the storage unit 12. (2) The controller 13 executes a known segmentation process and identifies a contour portion of the whole brain area. (3) The controller 13 acquires a pixel value of a pixel in the identified contour portion. (4) The controller 13 allows the storage unit 12 to store data on the pixel value of the acquired pixel.

Subsequently, the controller 13 reads preset reference information stored in the storage unit 12 (Activity A240). Here, the reference information is information where a type of a medical image, a pixel value, and a material are associated with each other, and one material is associated with one pixel value. The type of the medical image is an MRI image in the present embodiment. Therefore, the reference information is two or more templates supporting MRI images. A material means, according to the invention, is a parameter of at least one of albedo (reflectivity to each of RGB (red, green, and blue) light), reflectance (metallic characteristic), roughness.

Subsequently, the controller 13 executes a setting step (Activity A250). In the setting step, based on the type of the medical image (MRI image in the present embodiment), a pixel value of a pixel in the contour portion of the whole brain area included in the MRI image 212, and the preset reference information, the setting unit 133 sets material information representing a material of the brain for the contour portion of the whole brain area included in the MRI image 212.

In other words, Activity A250 includes, for example, the following five steps of information processing. (1) The controller 13 reads data stored in the storage unit 12 and representing that the MRI image 212 is an image captured by a magnetic resonance imaging apparatus. (2) The controller 13 reads the data on the whole brain area in the extracted MRI image 212 stored in the storage unit 12. (3) The controller 13 executes the setting process on the reference information read in activity A240 and the data read in (1) and (2) above. (4) The controller 13 sets the material information for the contour portion of the whole brain area in the MRI image 212. (5) The controller 13 allows the storage unit 12 to store the material information.

Subsequently, the controller 13 executes a reconstruction step (Activity A260). In the reconstruction step, the reconstruction unit 134 reconstructs the plurality of MRI images 222 and generates first data including the material information on the brain. That is, the controller 13 generates first data including the material information on the brain by reading the plurality of MRI images 222 and the material information stored in the storage unit 12 and executing a reconstruction process. The controller 13 allows the storage unit 12 to store the generated first data.

Subsequently, the controller 13 executes a reconstruction step (Activity A270). In the reconstruction step, the reconstruction unit 134 reconstructs the plurality of CT images 220 and generates second data including a shape of the brain. That is, the controller 13 generates the second data including the shape of the brain by reading the plurality of CT images 220 stored in the storage unit 12 and executing a reconstruction process. The controller 13 allows the storage unit 12 to store the generated second data.

Subsequently, the controller 13 executes a correction step (Activity A280). In the correction step, the correction unit 135 may execute a correction process on at least one of the first data and the second data, and in the present embodiment, the correction process is executed on both the first data and the second data. The controller 13 adjusts a coordinate position in the first data and a coordinate position in the second data by reading the first data and the second data stored in the storage unit 12 and executing the correction process. The controller 13 allows the storage unit 12 to store the first data and the second data after the correction. The correction process is not limited, but refers to, for example, translating, rotating, scaling, etc. the first data and second data by affine transformation, offset processing, or the like.

Here, a description will be given of the usefulness of using two different types of medical images in the present embodiment. The CT image 210 is an image acquired by X rays, and therefore is less likely to be distorted and is likely to represent bone shapes with high accuracy. However, in the CT image 210, contrasts of brains are insufficient, and therefore a shape of a brain is not represented with high accuracy and is represented as a rough shape of the brain. Thus, from the CT image 210, the rough shape of the brain can be acquired by extracting an area on an inner side of a skull.

On the other hand, the MRI image 212 is an image acquired by a magnetic field, and therefore texture information on a brain is likely to be represented with high contrast. However, the MRI image 212 is likely to be distorted, and when resolution is insufficient, shapes are not represented with high accuracy. In other words, due to medical resources, with the MRI image 212, it is relatively difficult to acquire an image having a resolution with which a shape of a brain can be represented with high accuracy. Therefore, it has been difficult to highly accurately represent shapes of brains only with the MRI image 212.

Since the CT image 210 represents the shape on the inner side of the skull, the second data represents a shape on slightly outer side of the surface of the brain. Therefore, the controller 13 executes the correction process on the second data so that pixels on a slightly inner side of the contour portion are acquired. The first data represents a distorted shape of the brain due to image distortion. Therefore, the controller 13 executes the correction process on the first data so as to correct the distortion.

In the present embodiment, the controller 13 extracts only the area on the inner side of the skull from the CT image 210. This allows the rough shape of the brain to be acquired. Thereafter, the controller 13 applies the MRI image 212 to the acquired rough shape of the brain so as to apply texture information to the rough shape of the brain and thereby can acquire a brain shape rich in information.

The MRI image 212 has property of having variation in an image distortion degree for each coordinate in a predetermined imaged area depending on an effect of a magnetic field and property of an object. Therefore, the controller 13 may execute the correction process on the first data while changing weighting depending on the coordinates in the first data. For example, the controller 13 may correct the distortion of the first data by applying a larger affine transformation matrix to coordinates on the outer side in the first data and a smaller affine transformation matrix to coordinates on the inner side in the first data.

Subsequently, the controller 13 executes an estimation step (Activity A290). In the estimation step, based on the material information set in Activity A250, the estimation unit 136 estimates shape information on a detailed shape of the brain. That is, the controller 13 estimates the shape information on the detailed shape of the brain by reading the material information stored in the storage unit 12 and executing an estimation process. The controller 13 allows the storage unit 12 to store the estimated shape information. The detailed shape of the brain means, for example, shapes of wrinkles, shapes of blood vessels, or the like. The CT image 210 and the MRI image 212 do not include information on uneven shapes of the brain. In the estimation process, the detailed shape is changed to uneven shapes depending on, for example, color information included in the material information. The shape information represents, for example, information on unevenness of the shapes of wrinkles and blood vessels of the brain.

Subsequently, the controller 13 executes a reconstruction step (Activity A300). In the reconstruction step, based on the first data and the second data after the correction and the estimated shape information, the reconstruction unit 134 generates three-dimensional data 230 on the brain. That is, the controller 13 generates the three-dimensional data 230 on the brain by reading the first data and the second data after the correction and the estimated shape information respectively stored in the storage unit 12 and executing a generation process.

Subsequently, the controller 13 allows the display unit 14 to display the three-dimensional data 230 on the brain (Activity A310). That is, by reading the three-dimensional data 230 on the brain stored in the storage unit 12 and executing a display process, the controller 13 allows the display unit 14 to display the three-dimensional data 230 on the brain.

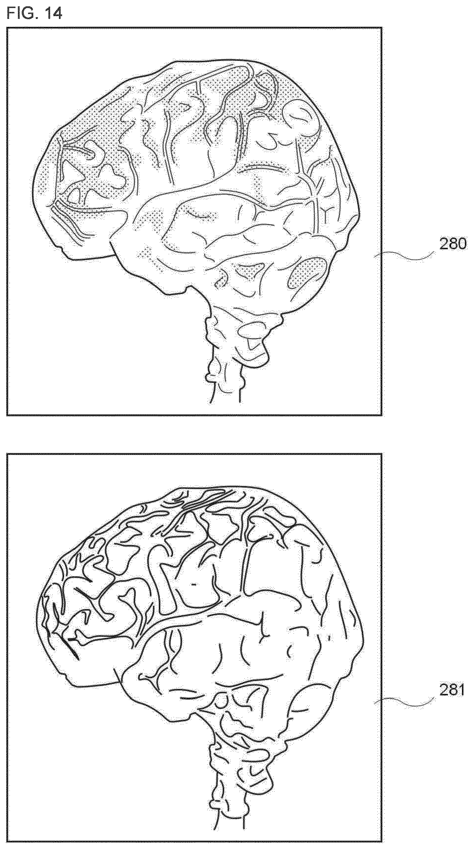

FIG. 14 is a diagram making a comparison between three-dimensional data without a correction process and three-dimensional data with a correction process. In an example 280 without the correction process, the microscopic shape can be visualized for the whole brain, but visibility is slightly reduced by shadows in some parts in the brain. In an example 281 with the correction process, there are almost no shadows in the whole brain, and visibility is better than in the example 280.

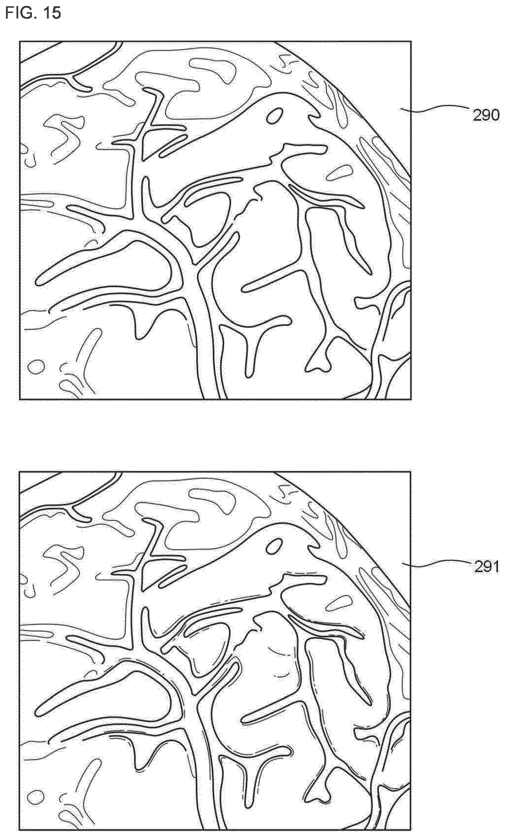

FIG. 15 is a diagram making a comparison between (partial) three-dimensional data without shape information based on a material and (partial) three-dimensional data with shape information based on a material. In an example 290 without shape information based on a material, microscopic shapes can be visualized for the whole brain, but uneven shapes of the brain surface cannot be sufficiently viewed. In an example 291 with shape information based on a material, visibility of uneven shapes of the brain surface is better than in the example 290.

In the present embodiment, sequential sectional images include a first medical image captured by a first medical image diagnosis apparatus and a second medical image captured by a second medical image diagnosis apparatus, the setting step sets, based on the first medical image, material information, the reconstruction step reconstructs the first medical image and generates first data including material information on an object, the reconstruction step reconstructs the second medical image and generates second data including a shape of the object, and the reconstruction step generates three-dimensional data on the object based on the first data and the second data. According to such an aspect, even when a type of an image with which a shape is easily formed (e.g., CT image) and a type of an image suitable to be referred to (e.g., MRI image) are different, by referring to a pixel value of a pixel in the image suitable to be referred to at a position corresponding to a position of a contour portion of a predetermined area in the image with which a shape is easily formed, microscopic shapes can be properly visualized.

In the present embodiment, the correction step executes a correction process on at least one of the first data and the second data, and based on the first data and the second data after the correction, the reconstruction step generates the three-dimensional data on the object. According to such an aspect, even when there is a positional shift between images (e.g., CT image and MRI image), distortion, a pixel value shift, or the like, microscopic shapes can be properly visualized.

In the present embodiment, the estimation step estimates, based on material information, shape information on a detailed shape of an object, and the reconstruction step generates, based on the shape information, three-dimensional data on the object. According to such an aspect, intricate structures such as wrinkles and blood vessels of brains can be clearly represented, which contributes to formulation of preoperative planning.

In the present embodiment, the first medical image diagnosis apparatus is a magnetic resonance imaging apparatus, and the second medical image diagnosis apparatus is an X-ray computed tomography apparatus. According to such an aspect, since three-dimensional data on an object can be generated by combining a CT image with which a shape is easily formed and an MRI image suitable to be referred to, microscopic shapes of the object can be visualized more suitably for characteristics of the object. In particular, when a shape of a brain is to be extracted from an MRI image, the MRI image needs to satisfy two requirements of (1) the type is proper (e.g., T1 weighted image, T2 weighted image, etc.) and (2) the resolution is high, and it may be difficult to acquire such an MRI image due to image-capturing cost and medical resources. On the other hand, according to the aspect of the present invention, microscopic shapes of a brain are easily visualized by using a CT image with which a shape on an inner side of a skull is easily formed.

### 5. Others

The following aspects may be adopted to the information processing apparatus 1 according to the present embodiments.
An aspect of the present embodiment may be SaaS (Software as a Service). In this case, the information processing apparatus 1 functions as a server on a cloud. The controller 13 may execute a setting process and a reconstruction process on a plurality of sequential sectional images of an object received by the communication unit 11 and transmits generated three-dimensional data via the communication unit 11.

As the first modification example, the type of the medical image is not limited and may be, for example, CT image, MRI image, PET image, or the like or may be T1 weighted image, T2 weighted image, Heavily T2 weighted image, time-of-flight (TOF) MRA image, FLAIR image, diffusion weighted image (DWI), susceptibility weighted image (SWI), MRV image, proton density weighted image, etc.

As the second modification example, the object is not particularly limited, and when the object is tissue or an organ of a human body, the object may be, for example, a bone, blood vessel, skin, an internal organ, an elbow joint, a knee joint, or the like.

As the third modification example, the predetermined area may be part of an object (e.g., a cerebellum area when the object is a brain), may be a whole object (e.g., a whole brain area when the object is a brain), or may be an area corresponding to an object (e.g., an area on an inner side of a skull when the object is a brain).

As the fourth modification example, the reconstruction unit 134 may determine, based on the material information instead of the third operation input made by the user, color viewed when the three-dimensional data is displayed and generate three-dimensional data. In this case, using a database in which material information and color information corresponding to the material information are organized, a learned model having performed machine learning using a relationship between material information and color information corresponding to the material information, or the like, the controller 13 may determine, based on the material information, the color viewed when the three-dimensional data is displayed.

According to the fourth modification example, even when the third operation input is not made by the user, microscopic shapes can be automatically represented with color.

As the fifth modification example, the setting unit 133 may select reference information depending on the predetermined area from two or more pieces of preset reference information not on the basis of the first operation input made by the user and may set material information on the basis of the selected reference information. In this case, the controller 13 may select the reference information depending on a predetermined area by using a database in which predetermined areas and reference information corresponding to the predetermined areas are organized, a learned model having performed machine learning on a relationship between the predetermined areas and reference information corresponding to the predetermined areas, or the like.

According to the fifth modification example, even when the first operation input is not made by the user, microscopic shapes can be automatically visualized more suitably for characteristics of the area.

As the sixth modification example, the controller 13 executes a writing (memory) process and a reading process on various types of data and various types of information into and from the storage unit 12, but processes are not limited to these, and the controller 13 may use, for example, a register or a cache memory in the controller 13 to execute information processing for each activity.

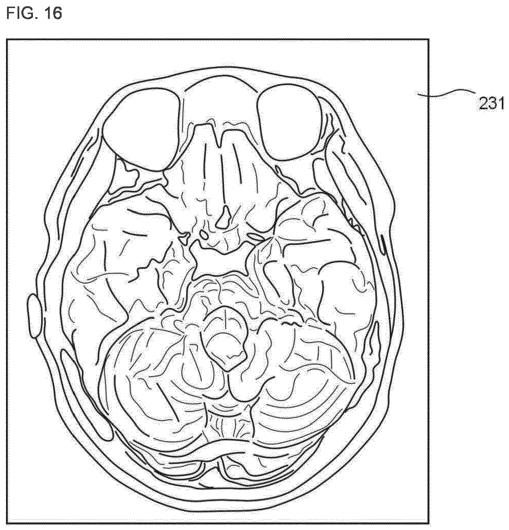

As the seventh modification example, the controller 13 is not limited to setting the material information for the contour portion of the predetermined area, but the controller 13 may set the material information for a portion other than the contour portion. FIG. 16 is a diagram illustrating a material application image 231 in which material information is set for a whole predetermined area (a whole brain in FIG. 16) in an arbitrary one of the plurality of MRI images 222.

According to the seventh modification example, it is possible to enrich information on an inner side of a predetermined area and to visualize microscopic shapes on the inner side of the predetermined area, which is useful when the inner side of the predetermined area is to be observed.

As the eighth modification example, a medical imaging diagnosis apparatus such as an X-ray computed tomography apparatus and a magnetic resonance imaging apparatus, may have functions of the information processing apparatus 1. In this case, the medical image diagnosis apparatus executes the information processing according to the present embodiment following each activity of the activity diagram illustrated in FIG. 3 or FIG. 12 and generates three-dimensional data on an object.

As the ninth modification example, the first medical image and the second medical image may be captured by the same type of apparatus. For example, the information processing apparatus 1 may generate first data and second data by reconstructing a first medical image and a second medical image captured by a magnetic resonance imaging apparatus and may generate, based on the first data and the second data after correction, three-dimensional data on an object.

As the tenth variation, in the reference information, one or more materials may be associated with one pixel value. In other words, in the reference information, two or more materials may be associated with one pixel value, or there may be two or more associated pairs of one pixel value and one material.

According to the tenth modification example, for example, even when pixel values are same between a pixel value in a frontal lobe part and a pixel value in an occipital lobe part in a cerebrum, different material information can be set for the frontal lobe part and for the occipital lobe part. In other words, even in a case where the pixel values are the same in different parts in a predetermined area, when, for example, special locations are different or the like, proper material information can be set in consideration of cases where proper materials are different.

Finally, various embodiments of the present invention have been described, but these are presented as examples and are not intended to limit the scope of the invention.

The present invention may be provided in each of the following aspects.

The information processing system, wherein the sequential sectional images are a medical image, and the object is predetermined tissue or organ of a human body.

The information processing system, wherein the controller executes the setting step of setting, based further on a type of the medical image, the material information, the reference information being information where the type of the medical image, a pixel value, and a material are associated with each other.

The information processing system wherein the sequential sectional images include a first medical image captured by a first medical image diagnosis apparatus and a second medical image captured by a second medical image diagnosis apparatus, and the controller is configured to execute the setting step of setting, based on the first medical image, the material information, the reconstruction step of reconstructing the first medical image and thereby generating first data including the material information on the object, the reconstruction step of reconstructing the second medical image and thereby generating second data including a shape of the object, and the reconstruction step of generating, based on the first data and the second data, the three-dimensional data.

The information processing system, wherein the controller is configured to execute a correction step of executing a correction process on at least one of the first data and the second data and the reconstruction step of generating, based on the first data and the second data after the correction process, the three-dimensional data.

The information processing system, wherein the controller is configured to execute an estimation step of estimating, based on the material information, shape information on a detailed shape of the object, and the reconstruction step of generating, based on the shape information, the three-dimensional data.

The information processing system, wherein the first medical image diagnosis apparatus is a magnetic resonance imaging apparatus, and the second medical image diagnosis apparatus is an X-ray computed tomography apparatus.

The information processing system, wherein the controller executes the reconstruction step of determining, based on the material information, color viewed when the three-dimensional data is displayed and thereby generating the three-dimensional data.

The information processing system, wherein in the reference information, one material is associated with one pixel value.

The information processing system, wherein the controller executes the setting step of setting, based on a pixel value of a pixel in a contour portion of the predetermined area and the reference information, the material information for the contour portion.

The information processing system, wherein in the setting step, the reference information is selected from two or more pieces of preset reference information depending on the predetermined area, and the material information is set based on the reference information.

The information processing system, wherein the controller is configured to execute a first receiving step of receiving first operation input made by a user with respect to the sequential sectional images, the first operation input including information selecting one from two or more pieces of preset reference information, and the setting step sets, based on the first operation input, the material information.

The information processing system, wherein the controller is configured to further execute a second receiving step of receiving second operation input made by a user with respect to the sequential sectional images, the second operation input including information specifying the predetermined area.

The information processing system, wherein the controller is configured to execute a third receiving step of receiving third operation input made by a user with respect to the sequential sectional images, the third operation input including information specifying color corresponding to the material information, and the reconstruction step of generating, based on the material information and the third operation input, the three-dimensional data.

A program allowing a computer to execute each step of the information processing system.

An information processing method comprising each step of the information processing system.

The present invention is not limited to those.

### [Description of Signs]

- 1: : Information processing apparatus
- 10: : Communication Bus
- 11: : Communication Unit
- 12: : Memory
- 13: : Controller
- 14: : Display unit
- 15: : Input unit
- 131: : Reading unit
- 132: : Receiving unit
- 133: : Setting unit
- 134: : Reconstruction unit
- 135: : Correction unit
- 136: : Estimation unit
- 210: : CT Image
- 211: : Contour Image
- 212: : MRI Image
- 213: : contour portion
- 220: : Multiple CT images
- 222: : plurality of MRI images
- 230: three-dimensional data
- 231: Material Application Images
- 242: : Area
- 252: : Area
- 300: :Setting screen
- 310: : three-dimensional data generation area
- 320: : Template Area
- 321: : Template
- 322: : Template
- 323: : Template
- 324: : Template
- 330: Material setting area
- 331: : Slider
- 332: : Slider
- 340: : Edit Area
- 341: : Addition button
- 342: : Clear button
- 350: : Auto button
- 360: : OK button
- 442: : Area
- 452: : Area

## Claims

1. An information processing system (1) comprising a controller (13) configured to execute each of following steps including:
a reading step (A110) of reading a plurality of sequential sectional images (222) of an object;
a setting step (A170) of setting, based on a pixel value of a pixel in a predetermined area included in the sequential sectional images (222) and preset reference information, material information representing a material of the object for the predetermined area, the reference information being information where a pixel value and a material are associated with each other, wherein the material is a parameter at least one of albedo, reflectance and roughness, the material information is information on the parameter; and
a reconstruction step (A190) of reconstructing the plurality of sequential sectional images (222) including the predetermined area for which the material information is set and thereby generating three-dimensional data (230) on the object.

2. The information processing system (1) according to claim 1, wherein:
the sequential sectional images (222) are a medical image, and
the object is predetermined tissue or organ of a human body.

3. The information processing system (1) according to claim 2, wherein the controller (13) executes the setting step (A170) of setting, based further on a type of the medical image, the material information, the reference information being information where the type of the medical image, a pixel value, and a material are associated with each other.

4. The information processing system (1) according to claim 2 or 3, wherein:
the sequential sectional images (222) include a first medical image (212) captured by a first medical image diagnosis apparatus and a second medical image (212) captured by a second medical image diagnosis apparatus; and
the controller (13) is configured to execute:
the setting step (A170) of setting, based on the first medical image (212), the material information;
the reconstruction step (A190) of reconstructing the first medical image (212) and thereby generating first data including the material information on the object;
the reconstruction step (A190) of reconstructing the second medical image (212) and thereby generating second data including a shape of the object; and
the reconstruction step (A190) of generating, based on the first data and the second data, the three-dimensional data (230).

5. The information processing system according to claim 4, wherein the controller (13) is configured to execute:
a correction step of executing a correction process on at least one of the first data and the second data; and
the reconstruction step (A190) of generating, based on the first data and the second data after the correction process, the three-dimensional data (230).

6. The information processing system according to claim 4 or 5, wherein the controller (13) is configured to execute:
an estimation step of estimating, based on the material information, shape information on a detailed shape of the object; and
the reconstruction step (A190) of generating, based on the shape information, the three-dimensional data (230).

7. The information processing system according to any one of claims 4 to 6, wherein:
the first medical image diagnosis apparatus is a magnetic resonance imaging apparatus, and
the second medical image diagnosis apparatus is an X-ray computed tomography apparatus.

8. The information processing system according to any one of claims 1 to 7, wherein in the reference information, one material is associated with one pixel value.

9. The information processing system according to any one of claims 1 to 8, wherein the controller (13) executes the setting step (A170) of setting, based on a pixel value of a pixel in a contour portion (213) of the predetermined area and the reference information, the material information for the contour portion (213).

10. The information processing system according to any one of claims 1 to 9, wherein in the setting step (A170,), the reference information is selected from two or more pieces of preset reference information depending on the predetermined area, and the material information is set based on the reference information.

11. The information processing system according to any one of claims 1 to 10, wherein the controller (13) is configured to execute:
a first receiving step (A140) of receiving first operation input made by a user with respect to the sequential sectional images (222), the first operation input including information selecting one from two or more pieces of preset reference information; and
the setting step (A170) sets, based on the first operation input, the material information.

12. The information processing system according to any one of claims 1 to 11, wherein the controller (13) is configured to further execute a second receiving step (A160) of receiving second operation input made by a user with respect to the sequential sectional images (222), the second operation input including information specifying the predetermined area.

13. The information processing system according to any one of claims 1 to 12, wherein the controller (13) is configured to execute:
a third receiving step (A180) of receiving third operation input made by a user with respect to the sequential sectional images (222), the third operation input including information specifying color corresponding to the material information; and
the reconstruction step (A190) of generating, based on the material information and the third operation input, the three-dimensional data (230).

14. A program allowing a computer to execute each step of the information processing system according to any one of claims 1 to 13.

15. A computer- implemented information processing method comprising each step of the information processing system according to any one of claims 1 to 13.

## Patentansprüche

1. Informationsverarbeitungssystem (1), umfassend ein Steuergerät (13), das dazu konfiguriert ist, jeden der folgenden Schritte auszuführen, die folgendes beinhalten:
einen Leseschritt (A110) zum Lesen einer Vielzahl von aufeinanderfolgenden Schnittbildern (222) eines Objekts;
einen Festlegungsschritt (A170) zum Festlegen, basierend auf einem Pixelwert eines Pixels in einem vorbestimmten Bereich, der in den aufeinanderfolgenden Schnittbildern (222) und voreingestellten Referenzinformationen enthalten ist, von Materialinformationen, die ein Material des Objekts für den vorbestimmten Bereich darstellen, wobei die Referenzinformationen Informationen sind, bei denen ein Pixelwert und ein Material einander zugeordnet sind, wobei das Material ein Parameter ist, der mindestens eines von Albedo, Reflexionsfaktor und Rauheit ist, wobei die Materialinformationen Informationen über den Parameter sind; und
einen Rekonstruktionsschritt (A190) zum Rekonstruieren der Vielzahl von aufeinanderfolgenden Schnittbildern (222), die den vorbestimmten Bereich beinhalten, für den die Materialinformationen festgelegt sind, und dadurch Generieren dreidimensionaler Daten (230) auf dem Objekt.

2. Informationsverarbeitungssystem (1) nach Anspruch 1, wobei;
die aufeinanderfolgenden Schnittbilder (222) ein medizinisches Bild sind und das Objekt vorbestimmtes Gewebe oder ein Organ eines menschlichen Körpers ist.

3. Informationsverarbeitungssystem (1) nach Anspruch 2, wobei das Steuergerät (13) den Festlegungsschritt (A170) zum Festlegen, basierend ferner auf einem Typ des medizinischen Bildes, der Materialinformationen ausführt, wobei die Referenzinformationen Informationen sind, bei denen der Typ des medizinischen Bildes, ein Pixelwert und ein Material einander zugeordnet sind.

4. Informationsverarbeitungssystem (1) nach Anspruch 2 oder 3, wobei: die aufeinanderfolgenden Schnittbilder (222) ein erstes medizinisches Bild (212), das von einem ersten medizinischen Bilddiagnosegerät aufgenommen wird, und ein zweites medizinisches Bild (212), das von einem zweiten medizinischen Bilddiagnosegerät aufgenommen wird, beinhalten; und
das Steuergerät (13) dazu konfiguriert ist, folgendes auszuführen:
den Festlegungsschritt (A170) zum Festlegen, basierend auf dem ersten medizinischen Bild (212), der Materialinformationen;
den Rekonstruktionsschritt (A190) zum Rekonstruieren des ersten medizinischen Bildes (212) und dadurch Generieren erster Daten, die die Materialinformationen auf dem Objekt beinhalten;
den Rekonstruktionsschritt (A190) zum Rekonstruieren des zweiten medizinischen Bildes (212) und dadurch Generieren zweiter Daten, die eine Form des Objekts beinhalten; und
den Rekonstruktionsschritt (A190) zum Generieren, basierend auf den ersten Daten und den zweiten Daten, der dreidimensionalen Daten (230).

5. Informationsverarbeitungssystem nach Anspruch 4, wobei das Steuergerät (13) dazu konfiguriert ist, folgendes auszuführen:
einen Korrekturschritt zum Ausführen eines Korrekturprozesses an mindestens einem von den ersten Daten und den zweiten Daten; und
den Rekonstruktionsschritt (A190) zum Generieren, basierend auf den ersten Daten und den zweiten Daten nach dem Korrekturprozess, der dreidimensionalen Daten (230).

6. Informationsverarbeitungssystem nach Anspruch 4 oder 5, wobei das Steuergerät (13) dazu konfiguriert ist, folgendes auszuführen:
einen Schätzschritt zum Schätzen, basierend auf den Materialinformationen, von Forminformationen zu einer detaillierten Form des Objekts; und
den Rekonstruktionsschritt (A190) zum Generieren, basierend auf den Forminformationen, der dreidimensionalen Daten (230).

7. Informationsverarbeitungssystem nach einem der Ansprüche 4 bis 6, wobei:
das erste medizinische Bilddiagnosegerät ein Magnetresonanzbildgebungsgerät ist, und
das zweite medizinische Bilddiagnosegerät ein Röntgencomputertomographiegerät ist.

8. Informationsverarbeitungssystem nach einem der Ansprüche 1 bis 7, wobei in den Referenzinformationen ein Material einem Pixelwert zugeordnet ist.

9. Informationsverarbeitungssystem nach einem der Ansprüche 1 bis 8, wobei das Steuergerät (13) den Festlegungsschritt (A170) zum Festlegen, basierend auf einem Pixelwert eines Pixels in einem Konturabschnitt (213) des vorbestimmten Bereichs und den Referenzinformationen, der Materialinformationen für den Konturabschnitt (213) ausführt.

10. Informationsverarbeitungssystem nach einem der Ansprüche 1 bis 9, wobei in dem Festlegungsschritt (A170) die Referenzinformationen aus zwei oder mehr Stücken voreingestellter Referenzinformationen abhängig von dem vorbestimmten Bereich ausgewählt werden und die Materialinformationen basierend auf den Referenzinformationen festgelegt werden.

11. Informationsverarbeitungssystem nach einem der Ansprüche 1 bis 10, wobei das Steuergerät (13) dazu konfiguriert ist, folgendes auszuführen:
einen ersten Empfangsschritt (A140) zum Empfangen einer ersten Bedienungseingabe, die von einem Benutzer in Bezug auf die aufeinanderfolgenden Schnittbilder (222) vorgenommen wird, wobei die erste Bedienungseingabe Informationen beinhaltet, die eines aus zwei oder mehr Stücken vorgegebener Referenzinformationen auswählen; und
der Festlegungsschritt (A170) legt, basierend auf der ersten Bedienungseingabe, die Materialinformationen fest.

12. Informationsverarbeitungssystem nach einem der Ansprüche 1 bis 11, wobei das Steuergerät (13) dazu konfiguriert ist, ferner einen zweiten Empfangsschritt (A160) zum Empfangen einer zweiten Bedienungseingabe, die von einem Benutzer in Bezug auf die aufeinanderfolgenden Schnittbilder (222) vorgenommen wird, auszuführen, wobei die zweite Bedienungseingabe Informationen beinhaltet, die den vorbestimmten Bereich spezifizieren.

13. Informationsverarbeitungssystem nach einem der Ansprüche 1 oder 12, wobei das Steuergerät (13) dazu konfiguriert ist, folgendes auszuführen:
einen dritten Empfangsschritt (A180) zum Empfangen einer dritten Bedienungseingabe, die von einem Benutzer in Bezug auf die aufeinanderfolgenden Schnittbilder (222) vorgenommen wird, wobei die dritte Bedienungseingabe Informationen beinhaltet, die eine Farbe spezifizieren, die den Materialinformationen entspricht; und
den Rekonstruktionsschritt (A190) zum Generieren, basierend auf den Materialinformationen und der dritten Bedienungseingabe, der dreidimensionalen Daten (230).

14. Programm, das es einem Computer ermöglicht, jeden Schritt des Informationsverarbeitungssystems nach einem der Ansprüche 1 bis 13 auszuführen.

15. Computerimplementiertes Informationsverarbeitungsverfahren, das jeden Schritt des Informationsverarbeitungssystems nach einem der Ansprüche 1 bis 13 umfasst.

## Revendications

1. Système de traitement d'informations (1) comprenant un dispositif de commande (13) configuré pour exécuter chacune des étapes suivantes comprenant :
une étape de lecture (A110) de lecture d'une pluralité d'images en coupe séquentielles (222) d'un objet ;
une étape de réglage (A170) de réglage, sur la base d'une valeur de pixel d'un pixel dans une zone prédéterminée comprise dans les images en coupe séquentielles (222) et d'informations de référence prédéfinies, d'informations de matériau représentant un matériau de l'objet pour la zone prédéterminée, les informations de référence étant des informations où une valeur de pixel et un matériau sont associés l'un à l'autre, dans lequel le matériau est un paramètre au moins l'un parmi l'albédo, la réflectance et la rugosité, les informations de matériau sont des informations sur le paramètre ; et
une étape de reconstruction (A190) de reconstruction de la pluralité d'images en coupe séquentielles (222) comprenant la zone prédéterminée pour laquelle les informations matérielles sont définies et ainsi de génération de données tridimensionnelles (230) sur l'objet.

2. Système de traitement d'informations (1) selon la revendication 1, dans lequel :
les images en coupe séquentielles (222) sont une image médicale, et l'objet est un tissu ou un organe prédéterminé d'un corps humain.

3. Système de traitement d'informations (1) selon la revendication 2, dans lequel le dispositif de commande (13) exécute l'étape de réglage (A170) de réglage, sur la base en outre d'un type de l'image médicale, des informations de matériau, les informations de référence étant des informations où le type de l'image médicale, une valeur de pixel et un matériau sont associés l'un à l'autre.

4. Système de traitement d'informations (1) selon la revendication 2 ou 3, dans lequel : les images en coupe séquentielles (222) comprennent une première image médicale (212) capturée par un premier appareil de diagnostic d'image médicale et une seconde image médicale (212) capturée par un second appareil de diagnostic d'image médicale ; et
le dispositif de commande (13) est configuré pour exécuter :
l'étape de réglage (A170) de réglage, sur la base de la première image médicale (212), des informations de matériau ;
l'étape de reconstruction (A190) de reconstruction de la première image médicale (212) et ainsi de génération de premières données comprenant les informations de matériau sur l'objet ;
l'étape de reconstruction (A190) de reconstruction de la seconde image médicale (212) et ainsi de génération de secondes données comprenant une forme de l'objet ; et
l'étape de reconstruction (A190) de génération, sur la base des premières données et des secondes données, des données tridimensionnelles (230).

5. Système de traitement d'informations selon la revendication 4, dans lequel le dispositif de commande (13) est configuré pour exécuter :
une étape de correction d'exécution d'un processus de correction sur au moins l'une des premières données et des secondes données ; et
l'étape de reconstruction (A190) de génération, sur la base des premières données et des secondes données après le processus de correction, des données tridimensionnelles (230).

6. Système de traitement d'informations selon la revendication 4 ou 5, dans lequel le dispositif de commande (13) est configuré pour exécuter :
une étape d'estimation d'estimation, sur la base des informations de matériau, d'informations de forme sur une forme détaillée de l'objet ; et
l'étape de reconstruction (A190) de génération, sur la base des informations de forme, des données tridimensionnelles (230).

7. Système de traitement d'informations selon l'une quelconque des revendications 4 à 6, dans lequel :
le premier appareil de diagnostic par imagerie médicale est un appareil d'imagerie par résonance magnétique, et
le second appareil de diagnostic d'image médicale est un appareil de tomodensitométrie à rayons X.

8. Système de traitement d'informations selon l'une quelconque des revendications 1 à 7, dans lequel, dans les informations de référence, un matériau est associé à une valeur de pixel.

9. Système de traitement d'informations selon l'une quelconque des revendications 1 à 8, dans lequel le dispositif de commande (13) exécute l'étape de réglage (A170) de réglage, sur la base d'une valeur de pixel d'un pixel dans une partie de contour (213) de la zone prédéterminée et des informations de référence, des informations de matériau pour la partie de contour (213).

10. Système de traitement d'informations selon l'une quelconque des revendications 1 à 9, dans lequel, à l'étape de réglage (A170), les informations de référence sont choisies parmi deux éléments d'informations de référence prédéfinis ou plus en fonction de la zone prédéterminée, et les informations de matériau sont réglées sur la base des informations de référence.

11. Système de traitement d'informations selon l'une quelconque des revendications 1 à 10, dans lequel le dispositif de commande (13) est configuré pour exécuter :
une première étape de réception (A140) de réception d'une première saisie d'opération effectuée par un utilisateur par rapport aux images en coupe séquentielles (222), la première saisie d'opération comprenant des informations choisissant un élément parmi deux éléments d'informations de référence prédéfinis ou plus ; et
l'étape de réglage (A170) règle, sur la base de la première saisie d'opération, les informations de matériau.

12. Système de traitement d'informations selon l'une quelconque des revendications 1 à 11, dans lequel le dispositif de commande (13) est configuré pour exécuter en outre une deuxième étape de réception (A160) de réception d'une deuxième saisie d'opération effectuée par un utilisateur par rapport aux images en coupe séquentielles (222), la deuxième saisie d'opération comprenant des informations spécifiant la zone prédéterminée.

13. Système de traitement d'informations selon l'une quelconque des revendications 1 à 12, dans lequel le dispositif de commande (13) est configuré pour exécuter :
une troisième étape de réception (A180) de réception d'une troisième saisie d'opération effectuée par un utilisateur par rapport aux images en coupe séquentielles (222), la troisième saisie d'opération comprenant des informations spécifiant une couleur correspondant aux informations de matériau ; et
l'étape de reconstruction (A190) de génération, sur la base des informations matérielles et de la troisième saisie d'opération, des données tridimensionnelles (230).

14. Programme permettant à un ordinateur d'exécuter chaque étape du système de traitement d'informations selon l'une quelconque des revendications 1 à 13.

15. Procédé de traitement d'informations mis en œuvre par ordinateur comprenant chaque étape du système de traitement d'informations selon l'une quelconque des revendications 1 à 13.
